(19) Europäisches Patentamt / European Patent Office / Office européen des brevets

(11) **EP 4 457 806 B1**

(12) **EUROPEAN PATENT SPECIFICATION**

(45) Date of publication and mention
of the grant of the patent:
**01.07.2026 Bulletin 2026/27**

(21) Application number: **23812203.0**

(22) Date of filing: **26.05.2023**

(51) International Patent Classification (IPC):
**G10L 25/66** (2013.01)   **A61B 5/087** (2006.01)
**G10L 25/18** (2013.01)   **G06N 3/0464** (2023.01)
**H04R 1/10** (2026.01)   **A61B 5/08** (2006.01)
**A61B 7/00** (2006.01)   **A61B 5/00** (2006.01)

(52) Cooperative Patent Classification (CPC):
**A61B 7/003; A61B 5/0816; A61B 5/6817;
A61B 5/7264; A61B 5/7267**

(86) International application number:
**PCT/KR2023/007284**

(87) International publication number:
**WO 2023/229429 (30.11.2023 Gazette 2023/48)**

(54) **SYSTEM AND METHOD FOR DEEP AUDIO SPECTRAL PROCESSING FOR RESPIRATION RATE AND DEPTH ESTIMATION USING SMART EARBUDS**

SYSTEM UND VERFAHREN ZUR TIEFENAUDIOSPEKTRALVERARBEITUNG ZUR ATEMFREQUENZ- UND TIEFENSCHÄTZUNG UNTER VERWENDUNG VON INTELLIGENTEN OHRHÖRERN

SYSTÈME ET PROCÉDÉ DE TRAITEMENT SPECTRAL AUDIO PROFOND POUR UNE ESTIMATION DE FRÉQUENCE ET D'AMPLITUDE RESPIRATOIRES UTILISANT DES ÉCOUTEURS INTELLIGENTS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.05.2022 US 202263346790 P
09.05.2023 US 202318314643**

(43) Date of publication of application:
**06.11.2024 Bulletin 2024/45**

(73) Proprietor: **Samsung Electronics Co., Ltd
Suwon-si, Gyeonggi-do 16677 (KR)**

(72) Inventors:
• **AHMED, Mohsin Yusuf
Mountain View, California 94043 (US)**
• **AHMED, Tousif
Mountain View, California 94043 (US)**
• **RAHMAN, Md Mahbubur
Mountain View, California 94043 (US)**
• **NEMATIHOSSEINABADI, Ebrahim
Mountain View, California 94043 (US)**
• **RASHID, Nafiul
Mountain View, California 94043 (US)**
• **KUANG, Jilong
Mountain View, California 94043 (US)**
• **GAO, Jun
Mountain View, California 94043 (US)**

(74) Representative: **Gulde & Partner
Patent- und Rechtsanwaltskanzlei mbB
Berliner Freiheit 2
10785 Berlin (DE)**

(56) References cited:
WO-A1-2020/141999    WO-A1-2020/141999
US-A1- 2018 220 904    US-A1- 2019 042 981
US-A1- 2019 042 981    US-A1- 2021 045 693
US-A1- 2021 146 082    US-A1- 2022 054 039
US-A1- 2022 087 609

## Description

[Technical Field]

The disclosure relates generally to electronic health monitoring systems and processes and, for example, the disclosure relates to a system and method for deep audio spectral processing for respiration rate and depth estimation using smart earbuds.

[Background Art]

Respiration rate (breaths per minute) and respiration depth (such as shallow, normal, or deep) are important health vital signs. Passive, continuous, and unobtrusive monitoring of respiration rate and depth helps many health and fitness applications determine an indicator of underlying disease, a biomarker for cardiopulmonary arrest, sleep quality, and the like. US 2022/0054039 A1 discloses a method measuring and analyzing human breathing in which distinct phases of a user's breathing according to classification of acoustic data by an ML model trained to classify acoustic data according to a teacher-student architecture with motion data as training input. US 2019/042981 A1 discloses a method for customizing output data on an electronic device associated with a user based on biological data of the user. WO 2020/141999 A1 relates to breathing monitoring system for a person suffering from asthma. US 2021/146082 A1 discloses a method and an apparatus for controlling delivering of gas to a patient.

The above information is presented as background information only to assist with an understanding of the disclosure. No determination has been made, and no assertion is made, as to whether any of the above might be applicable as prior art with regard to the disclosure.

[Disclosure of Invention]

[Solution to Problem]

The disclosure provides a system and method for deep audio spectral processing for respiration rate and depth estimation using smart earbuds.

According to the invention, a method is provided as set out in the appended set of claims.

According to another aspect of the invention, an electronic device is provided as set out in the appended set of claims.

In an example embodiment, a non-transitory machine-readable medium contains instructions that when executed cause at least one processor of an electronic device to obtain at least one breathing audio sample of a user captured using earbuds worn by the user. The medium also contains instructions that when executed cause the at least one processor to convert the at least one breathing audio sample to a breathing spectrogram configured as an image. The medium further contains instructions that when executed cause the at least one processor to process the breathing spectrogram using a trained multi-task CNN to identify a breathing rate and a breathing depth of the user. In addition, the medium contains instructions that when executed cause the at least one processor to output the breathing rate and the breathing depth of the user.

To further illustrate the advantages and features of the disclosure, a more particular description will be rendered by reference to various example embodiments thereof, which is illustrated in the appended drawings. It is appreciated that these drawings depict only example embodiments of the disclosure and are therefore not to be considered limiting its scope. The disclosure will be described and explained with additional specificity and detail with reference to the accompanying drawings.

[Brief Description of Drawings]

The above and other aspects, features, and advantages of certain embodiments of the disclosure will be more apparent from the following detailed description, taken in conjunction with the accompanying drawings, wherein like characters represent like parts throughout the drawings, and in which:

FIGURE 1 illustrates an example network configuration including an electronic device according to embodiments of the disclosure;

FIGURE 2 illustrates an example architecture for deep audio spectral processing for respiration rate and depth estimation using smart earbuds according to embodiments of the disclosure;

FIGURE 3 illustrates an example process for multi-task learning with a hybrid regression-classification loss function according to embodiments of the disclosure;

FIGURE 4 illustrates an example image representation of a breathing spectrogram according to embodiments of the disclosure;

FIGURE 5 illustrates an example configuration of a convolutional neural network (CNN) according to embodiments of the disclosure;

FIGURES 6A, 6B, and 6C illustrate example breathing patterns of a person engaged in different activities of daily living (ADLs) according to embodiments of the disclosure;

FIGURE 7 illustrates an example architecture for training an ADL-specific CNN model pool according to embodiments of the disclosure;

FIGURE 8 illustrates an example process for training an ADL-specific CNN model pool according to embodiments of the disclosure;

FIGURE 9 illustrates an example process for ADL-aware respiration rate and depth estimation using an ADL-specific CNN model pool according to embodiments of the disclosure; and

FIGURE 10 illustrates an example method for deep audio spectral processing for respiration rate and depth estimation using smart earbuds according to embodiments of the disclosure.

Further, skilled artisans will appreciate that elements in the drawings are illustrated for simplicity and may not have necessarily been drawn to scale. For example, the flowcharts illustrate the method in terms of operations involved to help to improve understanding of aspects of the disclosure. Furthermore, in terms of the construction of the device, one or more components of the device may have been represented in the drawings by conventional symbols, and the drawings may show those specific details that are pertinent to understanding the various example embodiments of the disclosure so as not to obscure the drawings with details that may be readily apparent to those of ordinary skill in the art.

[Mode for the Invention]

It may be advantageous to set forth definitions of certain words and phrases used throughout this patent document. The term "couple" and its derivatives refer to any direct or indirect communication between two or more elements, whether or not those elements are in physical contact with one another. The terms "transmit," "receive," and "communicate," as well as derivatives thereof, encompass both direct and indirect communication. The terms "include" and "comprise," as well as derivatives thereof, mean inclusion without limitation. The term "or" is inclusive, meaning and/or. The phrase "associated with," as well as derivatives thereof, means to include, be included within, interconnect with, contain, be contained within, connect to or with, couple to or with, be communicable with, cooperate with, interleave, juxtapose, be proximate to, be bound to or with, have, have a property of, have a relationship to or with, or the like. The term "controller" means any device, system, or part thereof that controls at least one operation. Such a controller may be implemented in hardware or a combination of hardware and software and/or firmware. The functionality associated with any particular controller may be centralized or distributed, whether locally or remotely. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C.

Moreover, various functions described below can be implemented or supported by one or more computer programs, each of which is formed from computer readable program code and embodied in a computer readable medium. The terms "application" and "program" refer to one or more computer programs, software components, sets of instructions, procedures, functions, objects, classes, instances, related data, or a portion thereof adapted for implementation in a suitable computer readable program code. The phrase "computer readable program code" includes any type of computer code, including source code, object code, and executable code. The phrase "computer readable medium" includes any type of medium capable of being accessed by a computer, such as read only memory (ROM), random access memory (RAM), a hard disk drive, a compact disc (CD), a digital video disc (DVD), or any other type of memory. A "non-transitory" computer readable medium excludes wired, wireless, optical, or other communication links that transport transitory electrical or other signals. A non-transitory computer readable medium includes media where data can be permanently stored and media where data can be stored and later overwritten, such as a rewritable optical disc or an erasable memory device.

Definitions for other certain words and phrases are provided throughout this patent document. Those of ordinary skill in the art should understand that in many, if not most instances, such definitions apply to prior as well as future uses of such defined words and phrases.

As used here, terms and phrases such as "have," "may have," "include," or "may include" a feature (like a number, function, operation, or component such as a part) indicate the existence of the feature and do not exclude the existence of other features. Also, as used here, the phrases "A or B," "at least one of A and/or B," or "one or more of A and/or B" may include all possible combinations of A and B. For example, "A or B," "at least one of A and B," and "at least one of A or B" may indicate all of (1) including at least one A, (2) including at least one B, or (3) including at least one A and at least one B. Further, as used here, the terms "first" and "second" may modify various components regardless of importance and do not limit the components. These terms are only used to distinguish one component from another. For example, a first user device and a second user device may indicate different user devices from each other, regardless of the order or importance of the devices. A first component may be denoted a second component and vice versa.

It will be understood that, when an element (such as a first element) is referred to as being (operatively or communicatively) "coupled with/to" or "connected with/to" another element (such as a second element), it can be coupled or connected with/to the other element directly or via a third element. In contrast, it will be understood that, when an element (such as a first element) is referred to as being "directly coupled with/to" or "directly connected with/to" another element (such as a second element), no other element (such as a third element) intervenes between the element and the other element.

As used here, the phrase "configured (or set) to" may be interchangeably used with the phrases "suitable for," "having the capacity to," "designed to," "adapted to," "made to," or "capable of" depending on the circumstances. The phrase "configured (or set) to" does not essentially mean "specifically designed in hardware to." Rather, the phrase "configured to" may mean that a device can perform an operation together with another device or parts. For example, the phrase "processor configured (or set) to perform A, B, and C" may mean a generic-purpose processor (such as a CPU or application processor) that may perform the operations by executing one or more software programs stored in a memory device or a dedicated processor (such as an embedded processor) for performing the operations.

The terms and phrases as used here are provided merely to describe some embodiments of the disclosure but not to limit the scope of other embodiments of the disclosure. It is to be understood that the singular forms "a," "an," and "the" include plural references unless the context clearly dictates otherwise. All terms and phrases, including technical and scientific terms and phrases, used here have the same meanings as commonly understood by one of ordinary skill in the art to which the embodiments of the disclosure belong. It will be further understood that terms and phrases, such as those defined in commonly-used dictionaries, should be interpreted as having a meaning that is consistent with their meaning in the context of the relevant art and will not be interpreted in an idealized or overly formal sense unless expressly so defined here. In some cases, the terms and phrases defined here may be interpreted to exclude embodiments of the disclosure.

Examples of an "electronic device" according to embodiments of the disclosure may include at least one of a smartphone, a tablet personal computer (PC), a mobile phone, a video phone, an e-book reader, a desktop PC, a laptop computer, a netbook computer, a workstation, a personal digital assistant (PDA), a portable multimedia player (PMP), an MP3 player, a mobile medical device, a camera, or a wearable device (such as smart glasses, a head-mounted device (HMD), electronic clothes, an electronic bracelet, an electronic necklace, an electronic accessory, an electronic tattoo, a smart mirror, or a smart watch). Other examples of an electronic device include a smart home appliance. Examples of the smart home appliance may include at least one of a television, a digital video disc (DVD) player, an audio player, a refrigerator, an air conditioner, a cleaner, an oven, a microwave oven, a washer, a drier, an air cleaner, a set-top box, a home automation control panel, a security control panel, a TV box (such as SAMSUNG HOMESYNC, APPLETV, or GOOGLE TV), a smart speaker or speaker with an integrated digital assistant (such as SAMSUNG GALAXY HOME, APPLE HOMEPOD, or AMAZON ECHO), a gaming console (such as an XBOX, PLAYSTATION, or NINTENDO), an electronic dictionary, an electronic key, a camcorder, or an electronic picture frame. Still other examples of an electronic device include at least one of various medical devices (such as diverse portable medical measuring devices (like a blood sugar measuring device, a heartbeat measuring device, or a body temperature measuring device), a magnetic resource angiography (MRA) device, a magnetic resource imaging (MRI) device, a computed tomography (CT) device, an imaging device, or an ultrasonic device), a navigation device, a global positioning system (GPS) receiver, an event data recorder (EDR), a flight data recorder (FDR), an automotive infotainment device, a sailing electronic device (such as a sailing navigation device or a gyro compass), avionics, security devices, vehicular head units, industrial or home robots, automatic teller machines (ATMs), point of sales (POS) devices, or Internet of Things (IoT) devices (such as a bulb, various sensors, electric or gas meter, sprinkler, fire alarm, thermostat, street light, toaster, fitness equipment, hot water tank, heater, or boiler). Other examples of an electronic device include at least one part of a piece of furniture or building/structure, an electronic board, an electronic signature receiving device, a projector, or various measurement devices (such as devices for measuring water, electricity, gas, or electromagnetic waves). Note that, according to various embodiments of the disclosure, an electronic device may be one or a combination of the above-listed devices. According to some embodiments of the disclosure, the electronic device may be a flexible electronic device. The electronic device disclosed here is not limited to the above-listed devices and may include new electronic devices depending on the development of technology.

In the following description, electronic devices are described with reference to the accompanying drawings, according to various embodiments of the disclosure. As used here, the term "user" may denote a human or another device (such as an artificial intelligent electronic device) using the electronic device.

Definitions for other certain words and phrases may be provided throughout this patent document. Those of ordinary skill in the art should understand that in many if not most instances, such definitions apply to prior as well as future uses of such defined words and phrases.

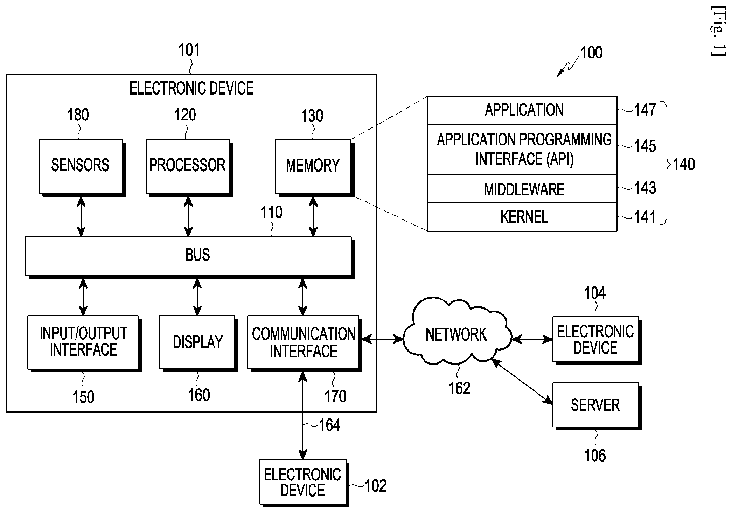

FIGURES 1 through 10, discussed below, and the various embodiments of the disclosure are described with reference to the accompanying drawings. However, it should be appreciated that the disclosure is not limited to these embodiments and all changes and/or equivalents or replacements thereto also belong to the scope of the disclosure.

As discussed above, respiration rate (breaths per minute) and respiration depth (such as shallow, normal, or deep) are important health vital signs. Passive, continuous, and unobtrusive monitoring of respiration rate and depth helps many health and fitness applications determine an indicator of underlying disease, a biomarker for cardiopulmonary arrest, sleep quality, and the like.

The proliferation of multi-modal, sensor-equipped, smart earbuds among the general population has made such earbuds suitable for in-ear health monitoring and a minimally-invasive, wearable device for accurate, continuous, and passive respiration rate and depth monitoring. For example, audio sensing from earbuds is an effective mechanism for estimating respiration rate and respiration depth. Earbuds, when worn, reside in close proximity to the respiratory tracts and may capture clear breathing sounds propagated internally through the human body. Various techniques use one or more inertial measurement unit (IMU) sensors (such as accelerometers, gyroscopes, and the like) in earbuds to detect respiration parameters. Respiration induces subtle head movements, which may be captured by the IMU sensors. However, IMU sensors are prone to error due to motion artifacts. Audio captured from the earbuds is not only resilient to motion artifacts but also inherently resilient to external ambient noise due to the phenomenon of "occlusion effect." Ambient noises like music mostly include high frequency components, and the occlusion effect in the earbuds suppresses these high frequency components and amplifies low frequency components where respiratory sounds reside.

Recent advancements in deep computer vision techniques have been demonstrated to be highly accurate in detecting various patterns and objects in images. However, prior techniques for inferring respiration parameters from earbud-captured audio data do not utilize an image representation of the audio data along with deep computer vision techniques. Such techniques may be useful in gaining more accurate deep image processing models for respiration parameters estimation.

Estimating multiple respiration parameters (like breathing rate and breathing depth) from an earbud-captured breathing audio signal is challenging. At a crude level, estimating N different respiration parameters from breathing audio may require N different machine learning models, which may be resource heavy in the form of more power, memory, and training time at a computing device. A model able to learn and infer multiple respiration parameters would be more efficient. Moreover, respiration parameters like respiration rate and depth are strongly dependent on the various activities of daily living (ADLs) in which a human subject may be engaged. However, prior techniques for inferring respiration parameters do not consider the human subject's activity state during model training or inferencing. This causes high variance in respiration parameter estimation performance at different activity states.

The disclosure provides various techniques for deep audio spectral processing for respiration rate and respiration depth estimation using smart earbuds. As described in more detail below, the systems and methods according to the invention as defined in claims 1 and 9 include deep image processing of earbud audio spectrograms using a single multi-task convolutional neural networks (CNN) in order to infer respiration parameters. The disclosed systems and methods may also include multi-task learning with a hybrid regression-classification loss function for simultaneous detection of respiration rate and respiration depth with a single model. In addition, the disclosed systems and methods may feature techniques for training a pool of ADL-aware multi-task CNN models for improved respiration rate and respiration depth detection during various activities. Note that while some of the embodiments discussed below are described in the context of use in consumer electronic devices (such as smart earbuds or smartphones), this is merely one example, and it will be understood that the principles of the disclosure may be implemented in any number of other suitable contexts and may use any suitable devices.

FIGURE 1 illustrates an example network configuration 100 including an electronic device according to an example embodiment of the disclosure. The embodiment of the network configuration 100 shown in FIGURE 1 is for illustration only.

According to embodiments of the disclosure, an electronic device 101 is included in the network configuration 100. The electronic device 101 includes a processor 120 and may include at least one of a bus 110, a memory 130, an input/output (I/O) interface 150, a display 160, a communication interface 170, or at least one sensor 180. In some embodiments, the electronic device 101 may exclude at least one of these components or may add at least one other component. The bus 110 includes a circuit for connecting the components 120 to 180 with one another and for transferring communications (such as control messages and/or data) between the components.

The processor 120 includes one or more processing devices, such as one or more microprocessors, microcontrollers, digital signal processors (DSPs), application specific integrated circuits (ASICs), or field programmable gate arrays (FPGAs). In some embodiments, the processor 120 includes one or more of a central processing unit (CPU), an application processor (AP), a communication processor (CP), or a graphics processor unit (GPU). The processor 120 is able to perform control on at least one of the other components of the electronic device 101 and/or perform an operation or data processing relating to communication or other functions. As described in more detail below, the processor 120 may perform one or more operations for deep audio spectral processing for respiration rate and depth estimation using smart earbuds.

The memory 130 may include a volatile and/or non-volatile memory. For example, the memory 130 may store commands or data related to at least one other component of the electronic device 101. According to embodiments of the disclosure, the memory 130 may store software and/or a program 140. The program 140 includes, for example, a kernel 141, middleware 143, an application programming interface (API) 145, and/or an application program (or "application") 147. At least a portion of the kernel 141, middleware 143, or API 145 may be denoted an operating system (OS).

The kernel 141 may control or manage system resources (such as the bus 110, processor 120, or memory 130) used to perform operations or functions implemented in other programs (such as the middleware 143, API 145, or application 147). The kernel 141 provides an interface that allows the middleware 143, the API 145, or the application 147 to access the individual components of the electronic device 101 to control or manage the system resources. The application 147 may support one or more functions for deep audio spectral processing for respiration rate and depth estimation using smart earbuds as discussed below. These functions may be performed by a single application or by multiple applications that each carry out one or more of these functions. The middleware 143 may function as a relay to allow the API 145 or the application 147 to communicate data with the kernel 141, for instance. A plurality of applications 147 may be provided. The middleware 143 is able to control work requests received from the applications 147, such as by allocating the priority of using the system resources of the electronic device 101 (like the bus 110, the processor 120, or the memory 130) to at least one of the plurality of applications 147. The API 145 is an interface allowing the application 147 to control functions provided from the kernel 141 or the middleware 143. For example, the API 145 includes at least one interface or function (such as a command) for filing control, window control, image processing, or text control.

The I/O interface 150 serves as an interface that may, for example, transfer commands or data input from a user or other external devices to other component(s) of the electronic device 101. The I/O interface 150 may also output commands or data received from other component(s) of the electronic device 101 to the user or the other external device.

The display 160 includes, for example, a liquid crystal display (LCD), a light emitting diode (LED) display, an organic light emitting diode (OLED) display, a quantum-dot light emitting diode (QLED) display, a microelectromechanical systems (MEMS) display, or an electronic paper display. The display 160 may also be a depth-aware display, such as a multi-focal display. The display 160 is able to display, for example, various contents (such as text, images, videos, icons, or symbols) to the user. The display 160 may include a touchscreen and may receive, for example, a touch, gesture, proximity, or hovering input using an electronic pen or a body portion of the user.

The communication interface 170, for example, is able to set up communication between the electronic device 101 and an external electronic device (such as a first electronic device 102, a second electronic device 104, or a server 106). For example, the communication interface 170 may be connected with a network 162 or 164 through wireless or wired communication to communicate with the external electronic device. The communication interface 170 may be a wired or wireless transceiver or any other component for transmitting and receiving signals.

The wireless communication is able to use at least one of, for example, WiFi, long term evolution (LTE), long term evolution-advanced (LTE-A), 5th generation wireless system (5G), millimeter-wave or 60 GHz wireless communication, Wireless USB, code division multiple access (CDMA), wideband code division multiple access (WCDMA), universal mobile telecommunication system (UMTS), wireless broadband (WiBro), or global system for mobile communication (GSM), as a communication protocol. The wired connection may include, for example, at least one of a universal serial bus (USB), high definition multimedia interface (HDMI), recommended standard 232 (RS-232), or plain old telephone service (POTS). The network 162 or 164 includes at least one communication network, such as a computer network (like a local area network (LAN) or wide area network (WAN)), Internet, or a telephone network.

The electronic device 101 may further include one or more sensors 180 that may meter a physical quantity or detect an activation state of the electronic device 101 and convert metered or detected information into an electrical signal. For example, one or more sensors 180 may include one or more cameras or other imaging sensors for capturing images of scenes. The sensor(s) 180 may also include one or more buttons for touch input, a gesture sensor, a gyroscope or gyro sensor, an air pressure sensor, a magnetic sensor or magnetometer, an acceleration sensor or accelerometer, a grip sensor, a proximity sensor, a color sensor (such as a red green blue (RGB) sensor), a bio-physical sensor, a temperature sensor, a humidity sensor, an illumination sensor, an ultraviolet (UV) sensor, an electromyography (EMG) sensor, an electroencephalogram (EEG) sensor, an electrocardiogram (ECG) sensor, an infrared (IR) sensor, an ultrasound sensor, an iris sensor, or a fingerprint sensor. The sensor(s) 180 may further include an inertial measurement unit, which may include one or more accelerometers, gyroscopes, and other components. In addition, the sensor(s) 180 may include a control circuit for controlling at least one of the sensors included here. Any of these sensor(s) 180 may be located within the electronic device 101.

In some embodiments, the electronic device 101 may be a wearable device or an electronic device-mountable wearable device (such as an HMD). For example, the electronic device 101 may represent an AR wearable device, such as a headset with a display panel or smart eyeglasses. In other embodiments, the first external electronic device 102 or the second external electronic device 104 may be a wearable device or an electronic device-mountable wearable device (such as an HMD). In those other embodiments, when the electronic device 101 is mounted in the first external electronic device 102 (such as the HMD), the electronic device 101 may communicate with the first external electronic device 102 through the communication interface 170. The electronic device 101 may be directly connected with the first external electronic device 102 to communicate with the first external electronic device 102 without involving a separate network.

The first and second external electronic devices 102 and 104 and the server 106 each may be a device of the same or a different type from the electronic device 101. According to certain embodiments of the disclosure, the server 106 includes a group of one or more servers. Also, according to certain embodiments of the disclosure, all or some of the operations executed on the electronic device 101 may be executed on another or multiple other electronic devices (such as the electronic devices 102 and 104 or server 106). Further, according to certain embodiments of the disclosure, when the electronic device 101 should perform some function or service automatically or at a request, the electronic device 101, instead of executing the function or service on its own or additionally, may request another device (such as electronic devices 102 and 104 or server 106) to perform at least some functions associated therewith. The other electronic device (such as electronic devices 102 and 104 or server 106) is able to execute the requested functions or additional functions and transfer a result of the execution to the electronic device 101. The electronic device 101 may provide a requested function or service by processing the received result as it is or additionally. To that end, a cloud computing, distributed computing, or client-server computing technique may be used, for example. While FIGURE 1 shows that the electronic device 101 includes the communication interface 170 to communicate with the second external electronic device 104 or server 106 via the network 162 or 164, the electronic device 101 may be independently operated without a separate communication function according to some embodiments of the disclosure.

The server 106 may include the same or similar components 110 to 180 as the electronic device 101 (or a suitable subset thereof). The server 106 may support to drive the electronic device 101 by performing at least one of operations (or functions) implemented on the electronic device 101. For example, the server 106 may include a processing module or processor that may support the processor 120 implemented in the electronic device 101. As described in more detail below, the server 106 may perform one or more operations to support techniques for deep audio spectral processing for respiration rate and depth estimation using smart earbuds.

Although FIGURE 1 illustrates an example of a network configuration 100 including an electronic device 101, various changes may be made to FIGURE 1. For example, the network configuration 100 could include any number of each component in any suitable arrangement. In general, computing and communication systems come in a wide variety of configurations, and FIGURE 1 does not limit the scope of the disclosure to any particular configuration. Also, while FIGURE 1 illustrates one operational environment in which various features disclosed in this patent document may be used, these features could be used in any other suitable system.

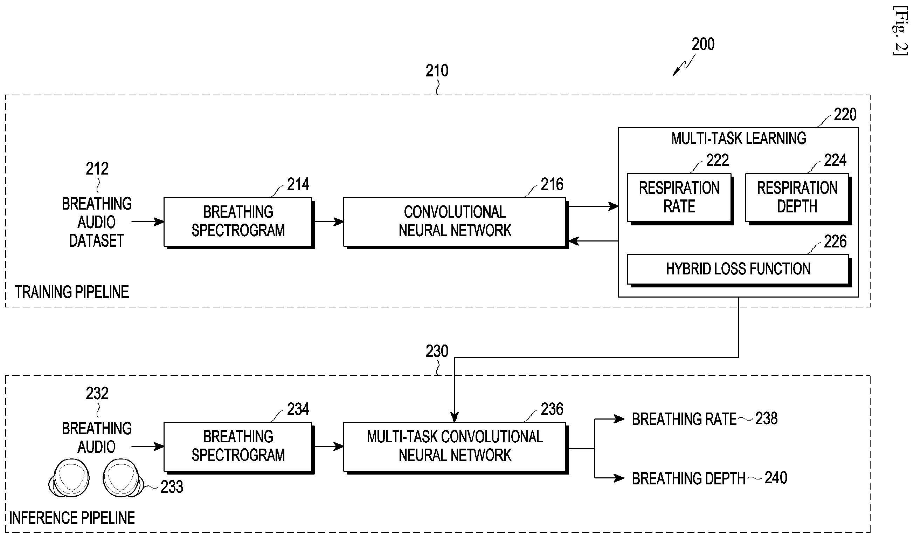

FIGURE 2 illustrates an example architecture 200 for deep audio spectral processing for respiration rate and depth estimation using smart earbuds according to an example embodiment of the disclosure. For ease of explanation, the architecture 200 is described as being implemented using one or more components of the network configuration 100 of FIGURE 1 described above, such as the electronic device 101. However, this is merely one example, and the architecture 200 could be implemented using any other suitable device(s) and in any other suitable system(s).

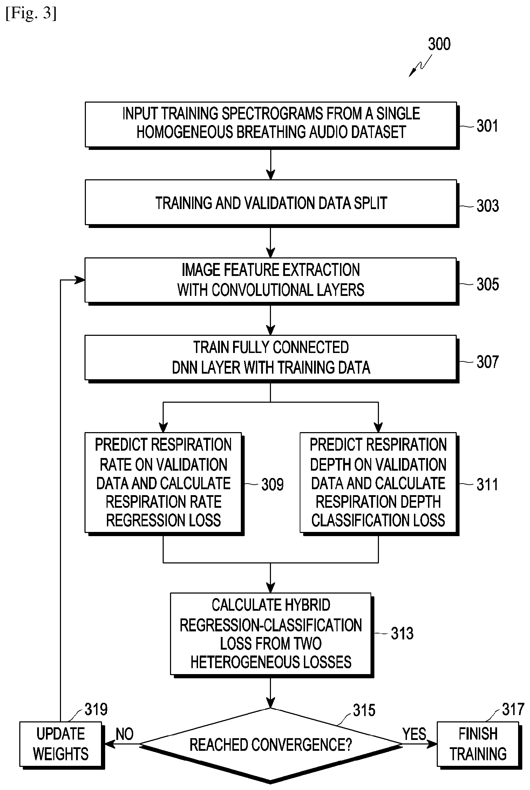

As shown in FIGURE 2, the architecture 200 includes a training pipeline 210 and an inference pipeline 230. In general, the training pipeline 210 includes or is otherwise associated with a breathing audio dataset 212, one or more breathing spectrograms 214, a convolutional neural network (CNN) 216, and a multi-task learning process 220. The multi-task learning process 220 is associated with a respiration rate 222, a respiration depth 224, and a hybrid loss function 226. A process performed using the training pipeline 210 will now be described in greater detail in conjunction with FIGURE 3.

FIGURE 3 illustrates an example process 300 for multi-task learning with a hybrid regression-classification loss function according to an example embodiment of the disclosure. For ease of explanation, the process 300 is described as being performed using one or more components of the network configuration 100 of FIGURE 1 described above (such as the electronic device 101) and the training pipeline 210 of FIGURE 2. However, this is merely one example, and the process 300 could be performed using any other suitable device(s) and in any other suitable system(s).

As shown in FIGURE 3, at operation 301, the electronic device 101 obtains breathing spectrograms 214 from the breathing audio dataset 212. The breathing audio dataset 212 includes multiple earbud breathing audio training samples that are annotated with breathing rate and breathing depth information. In operation 301, the earbud breathing audio training samples in the breathing audio dataset 212 are converted into the breathing spectrograms 214, which capture the inhalation and exhalation energy signatures in the time-frequency domain. The breathing spectrograms 214 are formatted as images and may be considered visual representations of breathing audio signals in the time-frequency domain. Each breathing spectrogram 214 is annotated with a breathing rate and breathing depth. As discussed below, the breathing spectrograms 214 are used to train the CNN 216 using the multi-task learning process 220. CNNs have been widely successful in detecting objects and patterns in images.

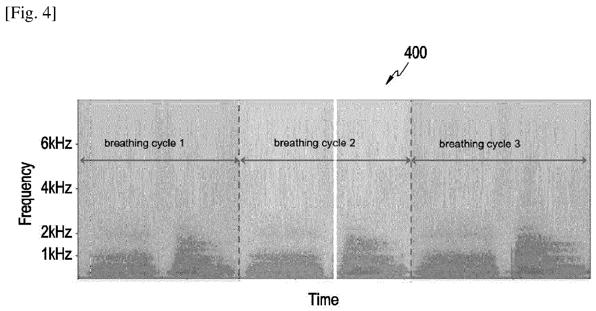

FIGURE 4 illustrates an example image representation 400 of a breathing spectrogram 214 according to an example embodiment of the disclosure.

As shown in FIGURE 4, the image representation 400 shows the energy level of the signal at different frequency bands and different times. Energy level at a frequency may be depicted by color, color intensity, or other indicator. In some embodiments, the breathing spectrograms 214 are generated by taking a short-time Fourier transform (STFT) with small frame size and stride (such as about 10 ms to about 50 ms) to obtain the power spectrum. Also, in some embodiments, the breathing spectrograms 214 include one or more mel-spectrograms. A mel-spectrogram is a type of spectrogram that is generated on the Mel frequency scale rather than the standard linear frequency scale. The Mel scale is a non-linear mapping of frequency to perceived loudness, which approximates the way the human auditory system processes sound.

Returning to FIGURE 3, at operation 303, the electronic device 101 performs a training and validation data split. Here, the electronic device 101 selects a portion of the training data (such as the breathing spectrograms 214) for training and selects a different portion of the training data for validation of the training. In some embodiments, approximately eighty percent of the training data may be selected for training, and the other approximately twenty percent may be selected for validation. In other embodiments, the percentages may be different.

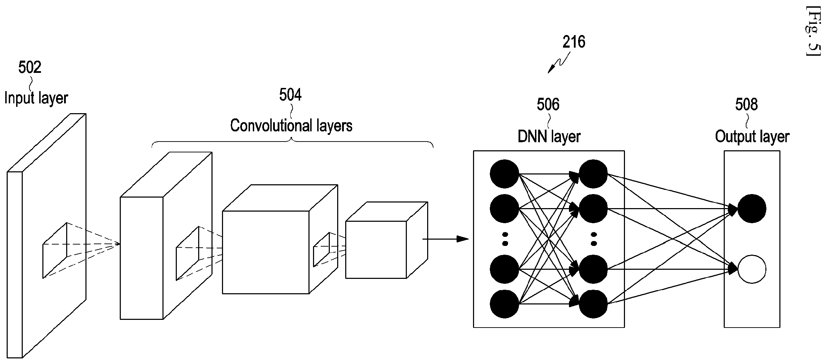

At operation 305, the electronic device 101 extracts image features from the breathing spectrograms 214 using convolutional layers of the CNN 216. FIGURE 5 illustrates an example configuration of the CNN 216 according to the disclosure. As shown in FIGURE 5, the CNN 216 includes an input layer 502, multiple convolutional layers 504, a deep neural network (DNN) layer 506, and an output layer 508. Here, the input layer 502 receives the breathing spectrograms 214 as input to the CNN 216. The convolutional layers 504 extract image features from the breathing spectrograms 214. The convolutional layers 504 may include any suitable type(s) and number of layers for extracting image features from one or more images.

At operation 307, the electronic device 101 trains the DNN layer 506 of the CNN 216 with training data, which may include the breathing audio dataset 212. The DNN layer 506 is a fully-connected layer that follows the convolutional layers 504.

The training in operation 307 is followed by two parallel predictions in operations 309 and 311. At operation 309, the electronic device 101 predicts a respiration rate 222 on the validation data selected in operation 303 and calculates a respiration rate regression loss. In this example, estimation of the respiration rate 222 is a regression task and has a regression loss function ($L_{regression}$). The electronic device 101 may use any suitable regression task and regression loss function, such as concordance correlation coefficient (CCC), mean squared error (MSE), mean absolute error (MAE), or the like. At operation 311, the electronic device 101 predicts a respiration depth 224 on the validation data and calculates a respiration depth classification loss. In this example, estimation of the respiration depth 224 is a classification task having a categorical cross-entropy loss function ($L_{classification}$).

At operation 313, the electronic device 101 calculates a hybrid loss function 226, which is a hybrid regression-classification loss that includes the two heterogeneous loss functions $L_{regression}$ and $L_{classification}$. In some embodiments, the electronic device 101 may calculate the hybrid loss function 226 using the following equation.

$$L_{total} = \alpha L_{regression} + \beta L_{classification}$$

Here, $L_{total}$ represents the hybrid loss function 226, and $\alpha$ and $\beta$ are weights that may be set empirically before or during training. In some embodiments, the values for $\alpha$ and $\beta$ may be customized based on user needs. For example, if a user is more interested in respiration rate than respiration depth, $\alpha$ may be assigned to provide more weight than $\beta$ (or vice versa).

At operation 315, the electronic device 101 determines if the training has reached convergence. In some embodiments, the electronic device 101 may compare the hybrid loss function 226 to a threshold that corresponds to convergence. If convergence has been reached, training is complete, and the electronic device 101 is finished with training as indicated at operation 317. If convergence has not been reached, training is not complete, and the process 300 moves to operation 319. At operation 319, the electronic device 101 updates training weights and returns to operation 305 to perform another iteration of the training.

It should be noted that the multi-task learning process 300 differs from some typical training techniques. In some typical techniques, a DNN model is trained by using multiple datasets, and the trained DNN model generates multiple outputs corresponding to the multiple datasets (such as dataset 'A' contributes to output 'a', dataset 'B' contributes to output 'b', and the like). Using such techniques, there are also multiple distinct losses corresponding to the multiple outputs. In contrast, the multi-task learning process 300 uses a single breathing audio dataset 212 as an input to learning and generates two outputs (respiration rate 222 and respiration depth 224) from the single input dataset. This multi-task learning process 300 uses the CNN 216 to identify both the respiration rate 222 and the respiration depth 224 by the single model using multi-task learning. Both targets are learned together in the same model, and learning one target may improve learning on the other target (and vice-versa). Also, the process 300 combines a regression loss function (from the respiration rate 222) and a classification loss function (from the respiration depth 224) into a hybrid loss function 226, in contrast to the multiple distinct losses used in other training techniques.

Although FIGURE 3 illustrates one example of a process 300 for multi-task learning with a hybrid regression-classification loss function, various changes may be made to FIGURE 3. For example, while shown as a series of operations, various operations in FIGURE 3 could overlap, occur in parallel, occur in a different order, or occur any number of times. Although FIGURE 4 illustrates one example of an image representation 400 of a breathing spectrogram, various changes may be made to FIGURE 4. For instance, the contents of the image representation 400 are for illustration only and may easily vary based on the data being obtained and processed. Although FIGURE 5 illustrates one example configuration of a CNN 216, various changes may be made to FIGURE 5. For example, each element of the CNN 216 may have any suitable design and include any suitable number of sub-components.

Turning again to FIGURE 2, once the CNN 216 is trained using the training pipeline 210, the CNN 216 may be used as a multi-task CNN 236 in the inference pipeline 230 during runtime. For example, using the inference pipeline 230, the electronic device 101 obtains a breathing audio signal 232 of a user. The breathing audio signal 232 is captured using earbuds 233 (such as captured using the earbuds' microphone(s)) while the user is wearing the earbuds 233. The breathing audio signal 232 is converted into at least one breathing spectrogram 234. Like the breathing spectrogram 214, the breathing spectrogram 234 captures the inhalation and exhalation energy signatures in the time-frequency domain and represents a visual representation of the breathing audio signal 232. The breathing spectrogram 234 shows the energy level of the breathing audio signal 232 at different frequency bands and at different times. In some embodiments, the breathing spectrogram 234 may be a mel-spectrogram that is generated on the Mel frequency scale rather than the standard linear frequency scale. The electronic device 101 provides the breathing spectrogram 234 as input to the multi-task CNN 236. Using the multi-task CNN 236, the electronic device 101 outputs (infers) respiration parameters from the breathing audio signal 232, such as a breathing rate 238 of the user and a breathing depth 240 of the user.

Although FIGURE 2 illustrates one example of an architecture 200 for deep audio spectral processing for respiration rate and depth estimation using smart earbuds, various changes may be made to FIGURE 2. For example, while described as involving specific sequences of operations, various operations of the techniques described with respect to FIGURE 2 could overlap, occur in parallel, occur in a different order, or occur any number of times (including zero times). Also, the specific operations shown in FIGURE 2 are examples only, and other techniques could be used to perform each of the operations shown in FIGURE 2.

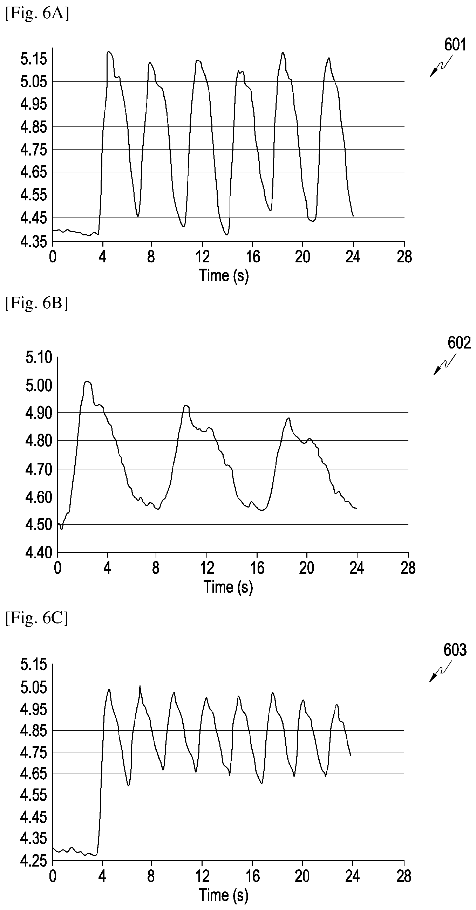

When a person engages in different activities of daily living (ADLs) (such as walking, running, sleeping, cycling, exercising, and the like), the ADLs may change the person's breathing behavior, breathing rate, and breathing depth. For example, FIGURES 6A through 6C illustrate example breathing patterns of a person engaged in different ADLs according to the disclosure.

In particular, FIGURE 6A shows a normal breathing pattern 601 during a static posture (such as sitting), FIGURE 6B shows a deep and slow breathing pattern 602 during sleeping, and FIGURE 6C shows a fast breathing pattern 603 during exercise. In this example, the breathing pattern 601 is shown as being 18 breaths per minute (BPM), the breathing pattern 602 is shown as being 9 BPM, and the breathing pattern 603 is shown as being 24 BPM (although each of these values may differ). Because different ADLs may result in significantly different breathing data, in some embodiments, breathing audio data may be collected for various ADLs, such as by using smart earbuds. Different datasets may include earbud-captured breathing audio for each of the different ADLs. In such embodiments, the CNN for deep audio spectral processing for respiration rate and depth estimation may be adapted to take into consideration multiple ADLs.

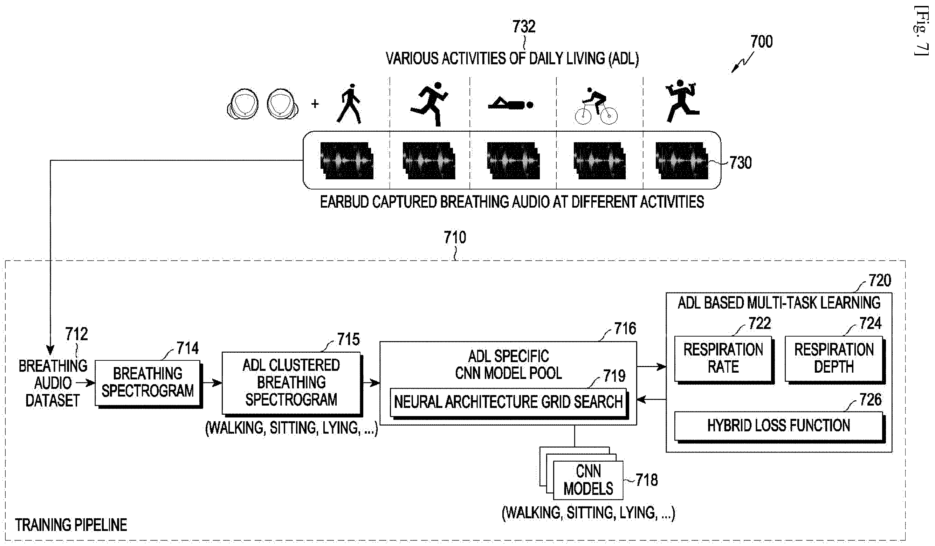

FIGURE 7 illustrates an example architecture 700 for training an ADL-specific CNN model pool according to embodiments of the disclosure. For ease of explanation, the architecture 700 is described as being implemented using one or more components of the network configuration 100 of FIGURE 1 described above, such as the electronic device 101. However, this is merely one example, and the architecture 700 could be implemented using any other suitable device(s) and in any other suitable system(s).

As shown in FIGURE 7, the architecture 700 includes multiple components that are the same as or similar to corresponding components of the architecture 200. The architecture 700 includes a training pipeline 710. In general, the training pipeline 710 includes or is associated with a breathing audio dataset 712, one or more breathing spectrograms 714, one or more ADL-clustered breathing spectrograms 715, an ADL-specific CNN model pool 716 containing multiple CNN models 718, and an ADL based multi-task learning process 720. The multi-task learning process 720 is associated with a respiration rate 722, a respiration depth 724, and a hybrid loss function 726. Here, the training pipeline 710 has the additional knowledge of which breathing audio sample belongs to which ADL class, along with the respiration rate and depth of the sample.

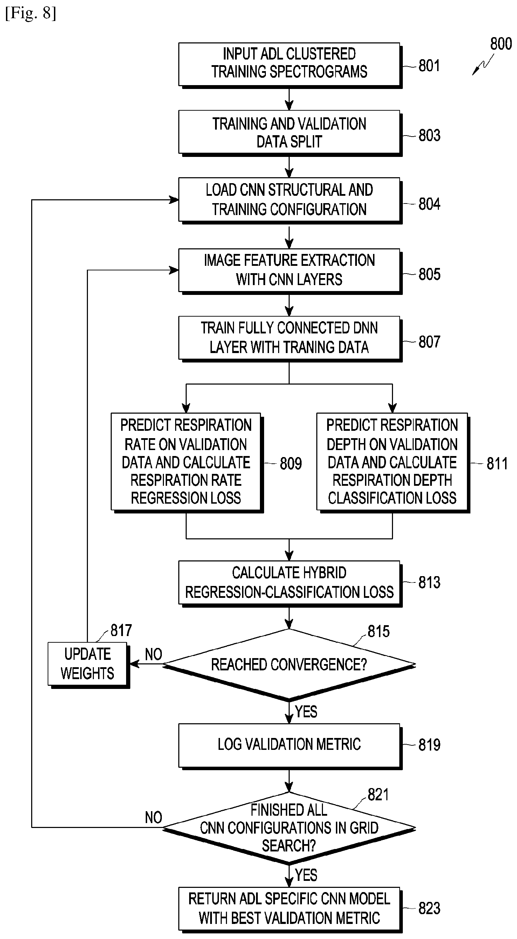

The breathing audio dataset 712 includes multiple earbud breathing audio training samples 730 that correspond to different ADLs 732. The breathing audio training samples 730 are annotated with breathing rate, breathing depth, and ADL information. The earbud breathing audio training samples 730 in the breathing audio dataset 712 are converted to breathing spectrograms 714, and the breathing spectrograms 714 are clustered based on ADL to form the ADL-clustered breathing spectrograms 715. For each cluster of breathing spectrograms 715, an ADL-specific CNN model 718 is trained. The result is a ADL-specific CNN model pool 716 that includes different CNN models 718 for the different ADLs 732. Each CNN model 718 is associated with a specific ADL 732. These ADL-specific CNN models 718 generally perform better in estimating respiration rate and depth than ADL-agnostic models. A process performed using the training pipeline 710 will now be described in greater detail in conjunction with FIGURE 8.

FIGURE 8 illustrates an example process 800 for training an ADL-specific CNN model pool according to embodiments of the disclosure.

As shown in FIGURE 8, at operation 801, the electronic device 101 obtains the ADL-clustered breathing spectrograms 715 from the breathing audio dataset 712. For example, the earbud breathing audio training samples 730 in the breathing audio dataset 712 may be converted to the breathing spectrograms 714, and the breathing spectrograms 714 may be clustered based on ADL to form the ADL-clustered breathing spectrograms 715. Each ADL-clustered breathing spectrogram 715 is used to train an ADL-specific CNN model 718.

At operation 803, the electronic device 101 performs a training and validation data split. Here, the electronic device 101 selects a portion of the training data (such as the ADL-clustered breathing spectrograms 715) for training and selects a different portion of the training data for validation of the training. In some embodiments, approximately eighty percent of the training data may be selected for training, and the other approximately twenty percent may be selected for validation. In other embodiments, the percentages may be different.

At operation 804, the electronic device 101 loads selected CNN structural and training configuration information associated with a particular configuration of the ADL-specific CNN models 718. Instead of the CNN models 718 all having a predefined CNN architecture with a fixed structure and parameters, each CNN model 718 may have a different CNN architecture. Therefore, a neural architecture grid search 719 may be performed in the process 800 to determine a suitable CNN architecture for each of the CNN models 718. The following CNN structural hyperparameters and grid search sample values are representative of architectural variables that might apply to the convolutional layers 504 of each CNN model 718: number of convolutional layers (such as 2, 3, 4, 5, ...); filter size (in pixels) (such as $2\times2$, $3\times3$, $4\times4$, $5\times5$, ...); number of filters (such as 16, 32, 64, 128, ...); stride size (such as 1 to the filter size); pooling type (such as max or avg); and activation function (such as rectified linear unit or "ReLU," sigmoid, Tanh, ...). The following DNN training hyperparameters and grid search sample values are representative of architectural variables that might apply to the DNN layer 506 of each CNN model 718: initial weights (such as random, Xavier, or He); learning rate (such as 0.1, 0.01, 0.001, ...); batch size (such as 32, 64, 128, ...); and optimizer (such as Adam, Gradient descent, RMSProp, ... ). In operation 804, the electronic device 101 loads one combination of each of these CNN and DNN hyperparameters and grid search values (such as number of convolutional layers = 3, pooling type = max, learning rate = 0.001, and so on) in order to test the suitability of the combination of values for the particular CNN model 718.

At operation 805, the electronic device 101 extracts image features from the ADL-clustered breathing spectrograms 715 using convolutional layers 504 of the particular CNN model 718.

At operation 807, the electronic device 101 trains the DNN layer 506 of the CNN model 718 with training data, which may include the breathing audio dataset 712. The training in operation 807 is followed by two parallel predictions in operations 809 and 811.

At operation 809, the electronic device 101 predicts a respiration rate 722 on the validation data selected in operation 803, and then calculates a respiration rate regression loss. In this example, estimation of the respiration rate 722 is a regression task and has a regression loss function ($L_{regression}$). The electronic device 101 may use any suitable regression task and regression loss function, such as concordance correlation coefficient (CCC), mean squared error (MSE), mean absolute error (MAE), or the like.

At operation 811, the electronic device 101 predicts a respiration depth 724 on the validation data and calculates a respiration depth classification loss. In this example, estimation of the respiration depth 724 is a classification task having a categorical cross-entropy loss function ($L_{classification}$).

At operation 813, the electronic device 101 calculates a hybrid loss function 726, which is a hybrid regression-classification loss that includes the two heterogeneous loss functions $L_{regression}$ and $L_{classification}$. In some embodiments, the electronic device 101 may calculate the hybrid loss function 726 using the following equation.

$$L_{total} = \alpha\, L_{regression} + \beta L_{classification}$$

Here, $L_{total}$ represents the hybrid loss function 226, and $\alpha$ and $\beta$ are weights that may be set empirically before or during training.

At operation 815, the electronic device 101 determines if the training has reached convergence. In some embodiments, the electronic device 101 may compare the hybrid loss function 726 to a threshold that corresponds to convergence. If convergence has not been reached for the current CNN model configuration, training is not complete, and the process 300 moves to operation 817.

At operation 817, the electronic device 101 updates the training weights and returns to operation 805 to perform another iteration of the training. If convergence has been reached in operation 815, training is complete, and the process 300 moves to operation 819.

At operation 819, the electronic device 101 logs one or more validation metrics indicating the results of the CNN model configuration (which includes the particular combination of CNN structural and training parameters selected in operation 804). It may be seen in FIGURE 8 that the loop including operations 805 through 817 may be performed multiple times for one particular configuration of CNN structural and training parameters.

At operation 821, the electronic device 101 determines if all CNN model configurations have been examined in the neural architecture grid search 719. If the electronic device 101 determines that not all CNN model configurations have been examined, the process 800 returns to operation 804, where the electronic device 101 selects another combination of CNN and DNN hyperparameters and grid search values. If the electronic device 101 determines that all CNN model configurations have been examined, the process 800 moves to operation 823.

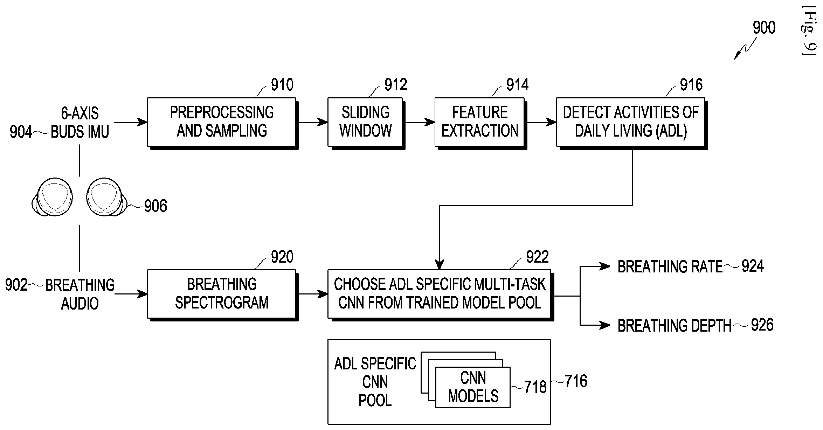

At operation 823, the electronic device 101 selects the configuration of the ADL-specific CNN model 718 with the best validation metric (as logged in operation 819). This provides the CNN configuration for each of the CNN models 718 in the ADL-specific CNN model pool 716. Following the process 800, the ADL-specific CNN model pool 716 is available for use in an inference (such as run-time) process, which is described below in conjunction with FIGURE 9.

Although FIGURES 6A through 6C illustrate examples of breathing patterns of a person engaged in different ADLs, various changes may be made to FIGURES 6A through 6C. For example, the breathing patterns shown here are examples only and may vary in different circumstances (such as for different users). Although FIGURES 7 and 8 illustrate one example of an architecture 700 and one example of a process 800 for training an ADL-specific CNN model pool, various changes may be made to FIGURES 7 and 8. For instance, while shown as a series of operations, various operations in FIGURES 7 and 8 could overlap, occur in parallel, occur in a different order, or occur any number of times. Also, the specific operations shown in FIGURES 7 and 8 are examples only, and other techniques could be used to perform each of the operations shown in FIGURES 7 and 8.

FIGURE 9 illustrates an example process 900 for ADL-aware respiration rate and depth estimation using an ADL-specific CNN model pool according to embodiments of the disclosure. The process 900 is an inference process that may occur, for instance, after the training process described in conjunction with FIGURES 7 and 8. During the process 900, a user's activity is detected in real-time (such as by using earbud six-axis IMU sensors or other IMU sensors like an accelerometer and gyroscope), and the detected activity-specific multitask CNN model is used to infer the respiration parameters from the breathing audio spectrogram. For ease of explanation, the process 900 is described as being implemented using one or more components of the network configuration 100 of FIGURE 1 described above, such as the electronic device 101. However, this is merely one example, and the process 900 could be implemented using any other suitable device(s) and in any other suitable system(s).

As shown in FIGURE 9, the electronic device 101 obtains a breathing audio signal 902 of the user. The breathing audio signal 902 is captured using earbuds 906 while the user is wearing the earbuds 906. The electronic device 101 converts the breathing audio signal 902 into a breathing spectrogram 920. As discussed above, the breathing spectrogram 920 captures the inhalation and exhalation energy signatures in the time-frequency domain and represents a visual representation of the breathing audio signal 902.

The electronic device 101 also obtains an IMU sensor data stream 904 from IMU sensors (such as accelerometer, gyroscope, and the like) in the earbuds 906. The IMU sensor data stream 904 may be obtained in parallel in the same temporal window as the breathing audio signal 902. At operation 910, the electronic device 101 performs preprocessing and sampling of the sensor data stream 904 at a specified sampling rate (such as 50 Hz). This could include any suitable preprocessing and sampling operations. At operation 912, the electronic device 101 defines a sliding window for the sensor data stream 904 (such as a five-second window with a one-second shift). At operation 914, the electronic device 101 extracts statistical features (such as mean, standard deviation, maximum, minimum, kurtosis, skewness, median, and the like) from the data stream window.

Based on extracted features, the electronic device 101 determines an ADL 916 in which the user in engaged at the time the breathing audio signal 902 and the sensor data stream 904 are obtained. The determined ADL 916 could include any of the following example ADLs: standing, sitting, lying, walking, jogging, vacuuming, climbing stairs, cycling, stretching, weightlifting, brushing teeth, washing hands, washing dishes, and the like. Note that these ADLs are examples only and may vary as needed or desired. In some embodiments, these specific ADLs may be grouped into broader ADL classes like posture (like sitting, standing, and lying down), moving (like walking, jogging, vacuuming, and climbing stairs), and stationary (like stretching and weight-lifting) if breathing parameter variations across more specific ADLs are not significant. Also, in some embodiments, the electronic device 101 uses a pretrained classifier to classify the extracted features into the ADL 916.

At operation 922, based on the determined ADL 916, the electronic device 101 selects an ADL-specific CNN model 718 from the ADL-specific CNN model pool 716. That is, the electronic device 101 selects a CNN model 718 that corresponds to the determined ADL 916 of the user. The electronic device 101 provides the breathing spectrogram 920 as input to the selected CNN model 718. Using the CNN model 718, the electronic device 101 outputs (infers) respiration parameters from the breathing audio signal 902, such as a breathing rate 924 of the user and a breathing depth 926 of the user.

Although FIGURE 9 illustrates one example of a process 900 for ADL-aware respiration rate and depth estimation using an ADL-specific CNN model pool, various changes may be made to FIGURE 9. For example, instead of obtaining IMU sensor data only from IMU sensors in the earbuds 906, the electronic device 101 may obtain IMU sensor data from IMU sensors in the earbuds 906 and in at least one other device, such as a smartwatch worn by the user. In such cases, IMU sensor data from the smartwatch may also be used to detect the ADL 916 in run-time. As another example, instead of training the CNN models 718 in the ADL-specific CNN model pool 716, one or more pretrained CNN models from other computer vision tasks (such as transfer learning) may be used to predict breathing rate and depth. In addition, while shown as a series of operations, various operations in FIGURE 9 could overlap, occur in parallel, occur in a different order, or occur any number of times.

Note that the operations and functions shown in or described with respect to FIGURES 2 through 9 may be implemented in an electronic device 101, 102, 104, server 106, or other device(s) in any suitable manner. For example, in some embodiments, the operations and functions shown in or described with respect to FIGURES 2 through 9 may be implemented or supported using one or more software applications or other software instructions that are executed by the processor 120 of the electronic device 101, 102, 104, server 106, or other device(s). In other embodiments, at least some of the operations and functions shown in or described with respect to FIGURES 2 through 9 may be implemented or supported using dedicated hardware components. In general, the operations and functions shown in or described with respect to FIGURES 2 through 9 may be performed using any suitable hardware or any suitable combination of hardware and software/firmware instructions.

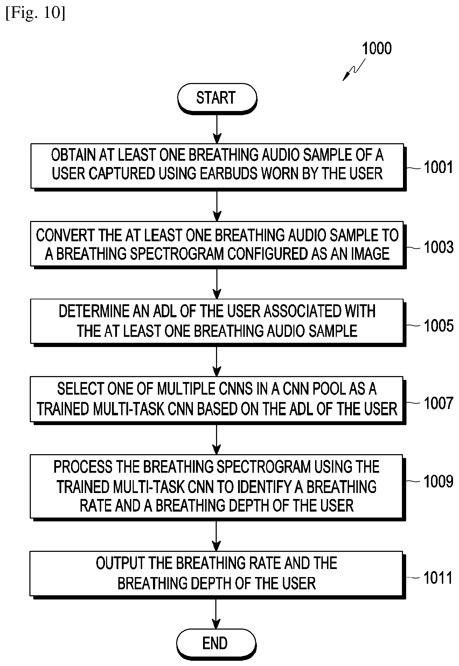

FIGURE 10 illustrates an example method 1000 for deep audio spectral processing for respiration rate and depth estimation using smart earbuds according to embodiments of the disclosure. For ease of explanation, the method 1000 shown in FIGURE 10 is described as involving the use of the architecture 700 shown in FIGURE 7, the processes 800 and 900 shown in FIGURES 8 and 9, and the electronic device 101 shown in FIGURE 1. However, the method 1000 shown in FIGURE 10 could be used with any other suitable architecture(s), process(es) and device(s).

As shown in FIGURE 10, at operation 1001, at least one breathing audio sample of a user is obtained. The at least one breathing audio sample is captured using earbuds worn by the user. This could include, for example, the electronic device 101 obtaining a breathing audio signal 902 of a user, which is captured using earbuds 906 worn by the user. The at least one breathing audio sample is converted to a breathing spectrogram configured as an image at operation 1003. This includes the electronic device 101 converting the breathing audio signal 902 to a breathing spectrogram 920.

An ADL of the user associated with the at least one breathing audio sample is determined at operation 1005. This could include, for example, the electronic device 101 determining an ADL 916 of the user based on an IMU sensor data stream 904 captured using IMU sensors in the earbuds 906. One of multiple CNNs in a CNN pool is selected as a trained multi-task CNN at operation 1007 based on the ADL of the user. This could include, for example, the electronic device 101 selecting one of the multiple CNN models 718 in the ADL-specific CNN model pool 716 as the trained multi-task CNN based on the determined ADL 916.

The breathing spectrogram is processed at operation 1009 using the trained multi-task CNN to identify a breathing rate and a breathing depth of the user. This could include, for example, the electronic device 101 processing the breathing spectrogram 920 using the selected CNN model 718 to identify a breathing rate 924 and a breathing depth 926 of the user. The breathing rate and the breathing depth of the user are output at operation 1011. This could include, for example, the electronic device 101 outputting the breathing rate 924 and the breathing depth 926. The breathing rate 924 and the breathing depth 926 could be shown on a display of the electronic device 101, output as audio via a speaker of the electronic device 101, transmitted to a data storage or another electronic device, or provided as any other suitable output.

Although FIGURE 10 illustrates one example of a method 1000 for deep audio spectral processing for respiration rate and depth estimation using smart earbuds, various changes may be made to FIGURE 10. For example, while shown as a series of operations, various operations in FIGURE 10 could overlap, occur in parallel, occur in a different order, or occur any number of times.

The embodiments described above may be advantageously implemented in multiple use cases. For example, in a first example use case, the disclosed embodiments may enable longitudinal respiration monitoring using earbuds. In some instances, there may be less overhead of use than with chest bands. Such monitoring using earbuds may include minimal overhead and may be minimally obtrusive. The monitoring using earbuds offers passive and continuous respiratory parameters measurement. Longitudinal respiration monitoring using earbuds may be more accurate than smartwatch-based solutions due to the earbuds' proximity to the respiratory tracts. Also, smartphone-based solutions may typically not be passive.

In a second example use case, the disclosed embodiments may enable remote patient respiratory monitoring using earbuds. This may be useful for patients with respiratory issues, such as irregular respiratory rate and agonal breathing. This may also be useful for elderly respiration monitoring and terminal patient respiration monitoring. In a third example use case, the disclosed embodiments may enable sleep respiration and sleep apnea monitoring using earbuds. In some instances, the disclosed embodiments may be used for overnight sleep respiration monitoring. The monitoring may be performed for detection of sleep apnea episodes, duration, and intensity. Such monitoring may be more accurate than monitoring with wrist-worn smartwatches. In a fourth example use case, the disclosed embodiments may enable activity-specific respiration monitoring using earbuds. In some instances, these embodiments may be useful for respiratory fitness measurement for athletes during training. These embodiments may be customized such that respiration sensing triggers automatically only for activities of interest.

In a fifth example use case, the disclosed embodiments may enable breathing biomarkers from the determined respiration rate and depth. Simultaneously detecting respiration rate and respiration depth offers the ability to extract several irregular breathing and breathing technique biomarkers, which could facilitate existing heath monitoring applications. As particular examples, the following biomarkers could be detected using respiration rate and depth:

Fast shallow breathing, also known as tachypnea: Tachypnea is a breathing pattern characterized by rapid breathing and reduced depth of each breath. It may be a symptom of various medical conditions, such as respiratory disorders (asthma, pneumonia, and COPD), heart disorders, metabolic disorders like diabetic ketoacidosis, anxiety, panic attacks, and drug and opioid overdose.

Fast deep breathing, also known as hyperventilation: Hyperventilation may be a symptom of panic attacks (which are episodes of intense fear and anxiety), asthma episodes, COPD, pulmonary fibrosis, heart disease, and metabolic disorders.

Slow shallow breathing: This may be a normal physiological response to relaxation or meditation. However, it may also be a symptom of various medical conditions, such as COPD, neuromuscular disorders like muscular dystrophy, myasthenia gravis, amyotrophic lateral sclerosis (ALS), opioid overdose, and sleep apnea.

Slow deep breathing: This may represent relaxation technique that involves taking slow, deep breaths in a controlled manner. The technique is often used to help reduce stress and anxiety, improve focus, and promote a sense of calmness.

The breathing biomarkers described above may be determined using the disclosed embodiments and provided to users as feedback on a health monitoring application (such as Samsung Health). Such breathing biomarkers may not be provided if the model generates a single reparatory metric. In addition, incorporating a single multi-task model ensures better accuracy of estimating respiratory rate and depth (as learning one target helps improves the other and vice versa), along with a smaller overall model size when compared to a solution using two single task models. In addition, the breathing biomarkers described above may facilitate or improve several existing and new health monitoring applications. Some of these applications will now be described.

A guided breathing exercise is a technique in which an individual follows the instructions of a guide app or a recording to control his or her breathing patterns. The exercise is designed to help individuals relax, reduce stress, and increase focus and awareness. During a guided breathing exercise, the guide typically directs the individual to take slow, deep breaths in through the nose and out through the mouth and to focus his or her attention on the sensation of the breath moving in and out of his or her body.

The importance of guided breathing exercises lies in their ability to activate the body's relaxation response, which may counteract the effects of stress on the body and mind. When a person experiences stress, the body's sympathetic nervous system is activated, leading to the release of stress hormones like cortisol and adrenaline. This may cause a range of physical and emotional symptoms, such as increased heart rate, shallow breathing, and feelings of anxiety and tension. By engaging in guided breathing exercises, one may activate the body's parasympathetic nervous system, which helps to reduce these stress-related symptoms and promote feelings of relaxation, calm, and well-being. This may have a range of benefits for both physical and mental health, such as improved sleep, reduced anxiety and depression, and lower blood pressure and heart rate.

Guided breathing exercises may be used in a variety of situations to promote relaxation, reduce stress, and increase focus and awareness. Some common use cases may include the following.

Stress reduction: Guided breathing exercises may be used to help reduce feelings of stress and anxiety. By activating the body's relaxation response, guided breathing exercises may help individuals feel more calm, centered, and in control.

Improved sleep: Practicing guided breathing exercises before bedtime may help promote relaxation and improve sleep quality. By slowing down the breathing and focusing the mind on the present moment, individuals may reduce racing thoughts and feel more at ease.

Increased focus: Guided breathing exercises may be used to increase focus and concentration, whether before a big meeting, presentation, or exam. By calming the mind and reducing distractions, individuals may improve their ability to focus and perform well.

Mindfulness: Guided breathing exercises may be used as part of a mindfulness practice to increase awareness and acceptance of the present moment. By focusing on the sensations of the breath, individuals may cultivate a sense of mindfulness and present-moment awareness.

Pain management: Guided breathing exercises may be used to help manage chronic pain or discomfort. By promoting relaxation and reducing tension in the body, individuals may reduce the perception of pain and improve their overall sense of wellbeing.

Real-time feedback may be very helpful during guided breathing exercises, as it may help individuals to stay focused and engaged and provide them with a better sense of their progress and performance in following the target breathing pattern. Some guided breathing exercise applications do not feature real-time feedback functions. Such applications may instruct the user to breathe at a certain rate and depth (such as with a visual animation) but may not know how well the user is performing in following the target breathing. The extracted raw respiration rate and respiration depth information obtained from the earbuds, along with the breathing biomarkers determined using the disclosed embodiments, may help provide real-time feedback for guided breathing exercises.

Guided breathing with real-time feedback using earbuds may be incorporated in various other functionalities and activities, such as meditation and yoga activities. Generally, meditation involves episodes of slow deep breathing, which may be detected using the disclosed embodiments for user feedback. Also, various stress detection applications may detect stress using smartphone- or smartwatch-captured heart rate and blood oxygen level. If high stress is detected, a stress management program may be proposed to the user by meditation using guided breathing exercises (generally slow deep breathings), where real-time breathing feedback may be provided to the user using the disclosed embodiments. Deep breathing techniques at guided postures may also play an important role in disease recovery, such as COVID-19 recovery. Such exercises include deep breathing while on one's back, while on one's stomach, while sitting, and while standing. The disclosed embodiments, along with high level breathing biomarkers, may detect deep breathing at various postures for real-time user feedback.

In some cases, the disclosed embodiments may produce respiration rate and depth outputs in real-time. In some example implementations, for instance, latency of audio-to-spectrogram generation during runtime may be between about 100 ms to about 120 ms, and latency for performing inference on the spectrogram using a chosen CNN model may be about 100 ms. In addition, the activity detection from IMU sensor signals may have a latency of about 50 ms (however, activity detection may occur in parallel with audio-to-spectrogram generation). As a result, after breathing audio is captured, these particular embodiments may produce a respiration rate and respiration breath output in about 200 ms to about 220 ms, which is essentially real-time operation.

In contrast, real-time biofeedback for different outputs using other monitoring techniques may not be synchronized with each other due to latency variations from different underlying algorithms/models, different window sizes, and different sensing modalities for different outputs. For example, one prior technique uses a ten-second window for respiration depth detection and a two-second window for phase detection in real-time. Also, respiration depth is detected from an IMU, while phase is detected from audio. Therefore, it would be harder for this technique to provide meaningful and synchronized combined biofeedback (such as inhale deeper and slower) to the user as the user performs a guided breathing exercise since feedback for depth is generated every ten seconds and feedback for phase is generated every two seconds. The disclosed embodiments are able to provide such meaningful combined synchronized biofeedback to the user in real-time due to use of a single sensing modality (audio), a single model (multi-task CNN), and a single window for all outputs (rate and depth).

Some other example use cases and related implementation details are presented below. In a sixth example use case, ADL-aware breathing inferences may be generated when a user is walking or jogging. Here, ADL detection may be performed (such as based on static posture, moving activity, stationary activity, or the like) using the earbuds' IMU sensors (such as accelerometer and gyroscope). The detected ADL class for walking or jogging may be "mooving activity." The earbuds' respiration audio may be converted into one or more spectrograms, and the respiration rate and respiration depth may be inferred from the spectrogram(s) using a selected ADL-aware respiration model. The outputs may include ADL-aware respiration rate and depth inference when the user is walking or jogging, which may be more accurate than ADL-agnostic models.

In a seventh example use case, ADL-aware breathing inferences may be generated when a user is exercising (such as lifting weights or stretching limbs). Here, ADL detection may be performed (such as based on static posture, moving activity, stationary activity, or the like) using the earbuds' IMU sensors (such as accelerometer and gyroscope). The detected ADL class for exercising may be "stationary activity." The earbuds' respiration audio may be converted into one or more spectrograms, and the respiration rate and respiration depth may be inferred from the spectrogram(s) using a selected ADL-aware respiration model. The outputs may include ADL-aware respiration rate and depth inference when the user is exercising, which may be more accurate than ADL-agnostic models.

In an eighth example use case, synchronized breathing during jogging or running may be encouraged. For example, a runner/jogger/trainer may set different synchronized breathing rate goals depending on training intensity, run length, and fitness. It may be helpful to check a runner's breathing rhythm occasionally because it may help monitor the running intensity. Some example breathing rhythms may include the following: 3 to 3 (the runner takes three strides while breathing in and three strides while breathing out); 2 to 2 (the runner takes two strides while breathing in and two strides while breathing out, which is a good training pace for most people and is often used during long runs and marathons); 2 to 1 (the runner takes two strides while breathing in and one stride while breathing out or vice versa); and 1 to 1 (the runner takes one stride while breathing in and one stride while breathing out).

An algorithm for synchronized breathing detection while jogging or running may be implemented as follows. A consecutive number of running/jogging strides (such as twelve strides) may be detected using the earbuds' IMU sensors, and time (t) may be measured for these strides. Respiration audio is captured using earbuds for those strides (within this time t), and user respiration rate is inferred using the disclosed embodiments. The user's respiration rate is compared with a target respiration rate based on the desired breathing rhythm. Thus, for a 1 to 1 breathing rhythm, the goal is six inhales and six exhales for twelve strides, and the target respiration rate is $(6{\times}60)/t$ BPM. For a 2 to 1 breathing rhythm, the goal is four inhales and four exhales for twelve strides, and the target respiration rate is $(4{\times}60)/t$ BPM. For a 2 to 2 goal, the goal is three inhales and three exhales for twelve strides, and the target respiration rate is $(3{\times}60)/t$ BPM. For a 3 to 3 goal, the goal is two inhales and two exhales for twelve strides, and the target respiration rate is $(2{\times}60)/t$ BPM. A recommendation may therefore be provided to the user in order to adjust his or her breathing if the user respiration rate mismatches the target respiration rate (such as when the user is breathing slower/faster) to synchronize with jogging.

As a particular example of this use case, assume a user is jogging and has a breathing rhythm goal of 2 to 2 (two strides per inhale and two strides per exhale). The user's device may count twelve strides using the earbuds' IMU and determine that the user took six seconds for the twelve strides. The target respiration rate may be calculated as $(3{\times}60)/6 = 30$ BPM. From breathing audio captured in earbuds within these six seconds, the user's respiration rate may be determined to be 24 BPM using the disclosed embodiments. Thus, the system may recommend that user breathe faster to meet the set goal (30 BPM) for synchronized breathing while jogging.

In a ninth example use case, synchronized breathing during exercise may be encouraged. For example, during exercise with repetitive limb motions (such as weightlifting), a user may want to synchronize his or her breathing pattern while lifting, such as inhaling before each lift and exhaling during each lift. Repetitive limb motions may be detected and counted accurately, such as by using IMU sensors in earbuds or a smartwatch. At the same time, earbud-captured audio may be used to detect respiration rate using the disclosed embodiments. A target respiration rate may therefore be compared with a detected respiration rate to provide a recommendation to the user to synchronize his or her breathing with the lifting exercises.

An algorithm for synchronized breathing detection while exercising may be implemented as follows. A consecutive number of limb movements (such as four limb movements) may be detected using IMU sensors in earbuds, a smartwatch, or both, and time (t) may be measured for these four limb movement cycles. Respiration audio may be captured using earbuds for the four limb movements (within this time t), and the user's respiration rate may be inferred using the disclosed embodiments. The user's respiration rate may be compared with a target respiration rate based on the user's goal. For example, for four limb movement cycles, the goal may be four inhales and four exhales, and the target respiration rate is $(4{\times}60)/t$ BPM. A recommendation may therefore be provided to the user in order to adjust his or her breathing if the user respiration rate mismatches the target respiration rate (such as when the user is breathing slower/faster) to synchronize with the exercise.

As a particular example of this use case, assume a user is weightlifting (bench pressing) with a breathing rhythm goal of an inhale before each lift and an exhale during each lift. The user's device may count four limb movement cycles using IMU sensors and determine that the user took eight seconds for these four limb movement cycles. The target respiration rate may be calculated as $(4{\times}60)/8 = 30$ BPM. From breathing audio captured in earbuds within these eight seconds, the user's respiration rate may be determined to be 40 BPM using the disclosed embodiments. Thus, the system may recommend that user breathe slower to meet the set goal (30 BPM) for synchronized breathing while weightlifting.

Note that the various use cases described above are non-limiting examples of how the disclosed embodiments may be used in various applications. The disclosed embodiments may be used in any other suitable applications or for any other suitable purposes and are not limited to these specific use cases.

While the disclosure has been illustrated and described with reference to various example embodiments, it will be understood that the various example embodiments are intended to be illustrative, not limiting. It will also be understood that any of the embodiment(s) described herein may be used in conjunction with any other embodiment(s) described herein.

## Claims

1. A method for deep audio spectral processing, the method comprising:

    obtaining at least one breathing audio sample (232) of a user captured using earbuds (233) worn by the user;
    converting the at least one breathing audio sample, (232) to a breathing spectrogram (234) configured as an image;
    processing the breathing spectrogram (234) using a single trained multi-task convolutional neural network, CNN, (236) to identify, from the breathing spectrogram (234), both a breathing rate (238) and a breathing depth (240) of the user; and

outputting the breathing rate (238) and the breathing depth (240) of the user.

2. The method of Claim 1, wherein the multi-task CNN (236) is trained using multi-task learning in which the multi-task CNN (236) is trained on multiple objective tasks in parallel, the multiple objective tasks comprising a regression task associated with a respiration rate (222) and a classification task associated with a respiration depth (224).

3. The method of Claim 2, wherein the multi-task learning uses a hybrid loss function (226) that combines a regression loss for the regression task and a classification loss for the classification task.

4. The method of claim 1, wherein the multi-task CNN (236) comprises one of multiple CNNs (718) in a CNN pool (716), each of the multiple CNNs (718) in the CNN pool (716) associated with a specific activity of daily living, ADL, (732).

5. The method of claim 4, wherein an architecture (700) of each of the multiple CNNs (718) in the CNN pool (716) is determined using a CNN neural architecture grid search (719) during training of the multiple CNNs (718).

6. The method of claim 4, further comprising, before processing the breathing spectrogram (920) using the trained multi-task CNN (236):

   determining an ADL (916) of the user associated with the at least one breathing audio sample (902); and
   selecting one of the multiple CNNs (718) in the CNN pool (716) as the trained multi-task CNN (236) based on the ADL (916) of the user.

7. The method of claim 6, wherein the ADL (916) of the user is determined using at least one of:

   motion data captured using the earbuds (233); and
   motion data captured using a smart watch worn by the user.

8. The method of claim 1, wherein the breathing spectrogram (234) comprises a mel-spectrogram.

9. An electronic device (101) for deep audio spectral processing, the electronic device comprising:
   at least one processor (120) configured to:

   obtain at least one breathing audio sample (232) of a user captured using earbuds (233) worn by the user;
   convert the at least one breathing audio sample (232) to a breathing spectrogram (234) configured as an image;
   process the breathing spectrogram (234) using a single trained multi-task convolutional neural network, CNN, (236) to identify, from the breathing spectrogram (234), both a breathing rate (238) and a breathing depth (240) of the user; and
   output the breathing rate (238) and the breathing depth (240) of the user.

10. The electronic device of Claim 9, wherein the multi-task CNN (236) is trained using multi-task learning in which the multi-task CNN (236) is trained on multiple objective tasks in parallel, the multiple objective tasks comprising a regression task associated with a respiration rate (222) and a classification task associated with a respiration depth (224).

11. The electronic device of Claim 10, wherein the multi-task learning uses a hybrid loss function (226) that combines a regression loss for the regression task and a classification loss for the classification task.

12. The electronic device of Claim 9, wherein the multi-task CNN (236) comprises one of multiple CNNs (718) in a CNN pool (716), each of the multiple CNNs (718) in the CNN pool (716) associated with a specific activity of daily living, ADL, (732).

13. The electronic device of claim 12, wherein an architecture of each of the multiple CNNs (718) in the CNN pool (716) is determined using a CNN neural architecture grid search (719) during training of the multiple CNNs (718).

14. The electronic device of claim 12, wherein the at least one processor is further configured, before processing the breathing spectrogram (234) using the trained multi-task CNN (236), to:

   determine an ADL (916) of the user associated with the at least one breathing audio sample; and
   select one of the multiple CNNs (718) in the CNN pool (716) as the trained multi-task CNN (236) based on the ADL (916) of the user.

15. The electronic device of Claim 14, wherein the at least one processing device is configured to determine the ADL (916) of the user using at least one of:

   motion data captured using the earbuds (233); and
   motion data captured using a smart watch worn by the user.

**Patentansprüche**

1. Verfahren zur tiefen audiospektralen Verarbeitung, wobei das Verfahren Folgendes umfasst:

   Erhalten mindestens einer Atem-Audioprobe (232) eines Benutzers, die unter Verwendung vom Benutzer getragener Ohrhörer (233) erfasst wird;
   Umwandeln der mindestens einen Atem-Audioprobe (232) in ein als Bild konfiguriertes Atemspektrogramm (234);
   Verarbeiten des Atemspektrogramms (234) unter Verwendung eines einzelnen trainierten Multitask-Convolutional-Neural-Netzwerks, CNN, (236), um aus dem Atemspektrogramm (234) sowohl eine Atemfrequenz (238) als auch eine Atemtiefe (240) des Benutzers zu identifizieren; und
   Ausgeben der Atemfrequenz (238) und der Atemtiefe (240) des Benutzers.

2. Verfahren nach Anspruch 1, wobei das Multitask-CNN (236) unter Verwendung von Multitask-Lernen trainiert wird, bei dem das Multitask-CNN (236) parallel auf mehrere objektive Aufgaben trainiert wird, wobei die mehreren objektiven Aufgaben eine mit einer Atemfrequenz (222) assoziierte Regressionsaufgabe und eine mit einer Atemtiefe (224) assoziierte Klassifizierungsaufgabe umfassen.

3. Verfahren nach Anspruch 2, wobei das Multitask-Lernen eine hybride Verlustfunktion (226) verwendet, die einen Regressionsverlust für die Regressionsaufgabe und einen Klassifizierungsverlust für die Klassifizierungsaufgabe kombiniert.

4. Verfahren nach Anspruch 1, wobei das Multitask-CNN (236) eines von mehreren CNNs (718) in einem CNN-Pool (716) umfasst, wobei jedes der mehreren CNNs (718) im CNN-Pool (716) mit einer spezifischen Aktivität des täglichen Lebens, ADL, (732) assoziiert ist.

5. Verfahren nach Anspruch 4, wobei eine Architektur (700) jedes der mehreren CNNs (718) im CNN-Pool (716) unter Verwendung einer CNN-Neuralarchitektur-Rastersuche (719) während des Trainings der mehreren CNNs (718) bestimmt wird.

6. Verfahren nach Anspruch 4, umfassend ferner vor der Verarbeitung des Atemspektrogramms (920) unter Verwendung des trainierten Multitask-CNN (236):

   Bestimmen einer ADL (916) des Benutzers, die mit der mindestens einen Atem-Audioprobe (902) assoziiert ist; und

   Auswählen eines der mehreren CNNs (718) im CNN-Pool (716) als das trainierte Multitask-CNN (236) auf der Grundlage der ADL (916) des Benutzers.

7. Verfahren nach Anspruch 6, wobei die ADL (916) des Benutzers unter Verwendung mindestens eines von Folgendem bestimmt wird:

   Bewegungsdaten, die unter Verwendung der Ohrhörer (233) erfasst werden; und
   Bewegungsdaten, die unter Verwendung einer vom Benutzer getragenen Smartwatch erfasst werden.

8. Verfahren nach Anspruch 1, wobei das Atemspektrogramm (234) ein Mel-Spektrogramm umfasst.

9. Elektronische Vorrichtung (101) zur tiefen audiospektralen Verarbeitung, wobei die elektronische Vorrichtung Folgendes umfasst:

   mindestens einen Prozessor (120), der dazu konfiguriert ist,
   mindestens eine Atem-Audioprobe (232) eines Benutzers zu erhalten, die unter Verwendung vom Benutzer getragener Ohrhörer (233) erfasst wird;
   die mindestens eine Atem-Audioprobe (232) in ein als Bild konfiguriertes AtemSpektrogramm (234) umzuwandeln;
   das Atemspektrogramm (234) unter Verwendung eines einzelnen trainierten Multitask-Convolutional-Neural-Netzwerks, CNN, (236) zu verarbeiten, um aus dem Atemspektrogramm (234) sowohl eine Atemfrequenz (238) als auch eine Atemtiefe (240) des Benutzers zu identifizieren; und
   die Atemfrequenz (238) und die Atemtiefe (240) des Benutzers auszugeben.

10. Elektronische Vorrichtung nach Anspruch 9, wobei das Multitask-CNN (236) unter Verwendung von Multitask-Lernen trainiert wird, bei dem das Multitask-CNN (236) parallel auf mehrere objektive Aufgaben trainiert wird, wobei die mehreren objektiven Aufgaben eine mit einer Atemfrequenz (222) assoziierte Regressionsaufgabe und eine mit einer Atemtiefe (224) assoziierte Klassifizierungsaufgabe umfassen.

11. Elektronische Vorrichtung nach Anspruch 10, wobei das Multitask-Lernen eine hybride Verlustfunktion (226) verwendet, die einen Regressionsverlust für die Regressionsaufgabe und einen Klassifizierungsverlust für die Klassifizierungsaufgabe kombiniert.

12. Elektronische Vorrichtung nach Anspruch 9, wobei das Multitasking-CNN (236) eines von mehreren CNNs (718) in einem CNN-Pool (716) umfasst, wobei jedes der mehreren CNNs (718) im CNN-Pool (716) mit einer spezifischen Aktivität des täglichen Lebens, ADL, (732) assoziiert ist.

13. Elektronische Vorrichtung nach Anspruch 12, wobei eine Architektur jedes der mehreren CNNs (718) im CNN-Pool (716) unter Verwendung einer CNN-Neuralarchitektur-Rastersuche (719) während des Trainings der mehreren CNNs (718) bestimmt wird.

14. Elektronische Vorrichtung nach Anspruch 12, wobei der mindestens eine Prozessor ferner dazu konfiguriert ist, vor der Verarbeitung des Atemspektrogramms (234) unter Verwendung des trainierten Multitask-CNN (236)

eine ADL (916) des Benutzers zu bestimmen, die mit der mindestens einen Atem-Audioprobe assoziiert ist; und
eines der mehreren CNNs (718) im CNN-Pool (716) als das trainierte Multitask-CNN (236) auf der Grundlage der ADL (916) des Benutzers auszuwählen.

15. Elektronische Vorrichtung nach Anspruch 14, wobei die mindestens eine Verarbeitungsvorrichtung dazu konfiguriert ist, die ADL (916) des Benutzers unter Verwendung mindestens eines von Folgendem zu bestimmen:

Bewegungsdaten, die unter Verwendung der Ohrhörer (233) erfasst werden; und
Bewegungsdaten, die unter Verwendung einer vom Benutzer getragenen Smartwatch erfasst werden.

**Revendications**

1. Procédé de traitement spectral audio profond, le procédé comprenant :

l'obtention d'au moins un échantillon audio de respiration (232) d'un utilisateur capturé en utilisant des écouteurs (233) portés par l'utilisateur ;
la conversion de l'au moins un échantillon audio de respiration, (232) en un spectrogramme de respiration (234) configuré comme une image ;
le traitement du spectrogramme de respiration (234) en utilisant un seul réseau neuronal convolutionnel multitâche entraîné, CNN, (236) pour identifier, à partir du spectrogramme de respiration (234), à la fois une fréquence expiratoire (238) et une amplitude expiratoire (240) de l'utilisateur ; et

la sortie de la fréquence expiratoire (238) et de l'amplitude expiratoire (240) de l'utilisateur.

2. Procédé de la revendication 1, dans lequel le CNN multitâche (236) est entraîné en utilisant un apprentissage multitâche dans lequel le CNN multitâche (236) est entraîné sur de multiples tâches objectives en parallèle, les multiples tâches objectives comprenant une tâche de régression associée à une fréquence respiratoire (222) et une tâche de classification associée à une amplitude respiratoire (224).

3. Procédé de la revendication 2, dans lequel l'apprentissage multitâche utilise une fonction de perte hybride (226) qui combine une perte de régression pour la tâche de régression et une perte de classification pour la tâche de classification.

4. Procédé de la revendication 1, dans lequel le CNN multitâche (236) comprend l'un de multiples CNN (718) dans un ensemble de CNN (716), chacun des multiples CNN (718) dans l'ensemble de CNN (716) étant associé à une activité spécifique de la vie quotidienne, ADL, (732).

5. Procédé de la revendication 4, dans lequel une architecture (700) de chacun des multiples CNN (718) dans l'ensemble de CNN (716) est déterminée en utilisant une recherche en grille d'architecture neuronale de CNN (719) pendant l'entraînement des multiples CNN (718).

6. Procédé de la revendication 4, comprenant en outre, avant le traitement du spectrogramme de respiration (920) en utilisant le CNN multitâche entraîné (236) :

la détermination d'une ADL (916) de l'utilisateur associée à l'au moins un échantillon audio de respiration (902) ; et
la sélection de l'un des multiples CNN (718) dans l'ensemble de CNN (716) comme le CNN multitâche entraîné (236) sur la base de l'ADL (916) de l'utilisateur.

7. Procédé de la revendication 6, dans lequel l'ADL (916) de l'utilisateur est déterminée en utilisant au moins l'une de :

données de mouvement capturées en utilisant les écouteurs (233) ; et
données de mouvement capturées en utilisant une montre intelligente portée par l'utilisateur.

8. Procédé de la revendication 1, dans lequel le spectrogramme de respiration (234) comprend un mel-spectrogramme.

9. Dispositif électronique (101) pour un traitement spectral audio profond, le dispositif électronique comprenant :

au moins un processeur (120) configuré pour :

obtenir au moins un échantillon audio de respiration (232) d'un utilisateur capturé en utilisant des écouteurs (233) portés par l'utilisateur ; convertir l'au moins un échantillon audio de respiration (232) en un spectrogramme de respiration (234) configuré comme une image ; traiter le spectrogramme de respiration (234) en utilisant un seul réseau neuronal convolutionnel multitâche entraîné, CNN, (236) pour identifier, à partir du spectrogramme de respiration (234), à la fois une fréquence expiratoire (238) et une amplitude expiratoire (240) de l'utilisateur ; et sortir la fréquence expiratoire (238) et l'amplitude expiratoire (240) de l'utilisateur.

10. Dispositif électronique de la revendication 9, dans lequel le CNN multitâche (236) est entraîné en utilisant un apprentissage multitâche dans lequel le CNN multitâche (236) est entraîné sur de multiples tâches objectives en parallèle, les multiples tâches objectives comprenant une tâche de régression associée à une fréquence respiratoire (222) et une tâche de classification associée à une amplitude respiratoire (224).

11. Dispositif électronique de la revendication 10, dans lequel l'apprentissage multitâche utilise une fonction de perte hybride (226) qui combine une perte de régression pour la tâche de régression et une perte de classification pour la tâche de classification.

12. Dispositif électronique de la revendication 9, dans lequel le CNN multitâche (236) comprend l'un de multiples CNN (718) dans un ensemble de CNN (716), chacun des multiples CNN (718) dans l'ensemble de CNN (716) étant associé à une activité spécifique de la vie quotidienne, ADL, (732).

13. Dispositif électronique de la revendication 12, dans lequel une architecture de chacun des multiples CNN (718) dans l'ensemble de CNN (716) est déterminée en utilisant une recherche en grille d'architecture neuronale de CNN (719) pendant l'entraînement des multiples CNN (718).

14. Dispositif électronique de la revendication 12, dans lequel l'au moins un processeur est en outre configuré, avant le traitement du spectrogramme de respiration (234) en utilisant le CNN multitâche entraîné (236), pour :

déterminer une ADL (916) de l'utilisateur associée à l'au moins un échantillon audio de respiration ; et

sélectionner l'un des multiples CNN (718) dans l'ensemble de CNN (716) comme le CNN multitâche entraîné (236) sur la base de l'ADL (916) de l'utilisateur.

15. Dispositif électronique de la revendication 14, dans lequel l'au moins un dispositif de traitement est configuré pour déterminer l'ADL (916) de l'utilisateur en utilisant au moins l'une de :

données de mouvement capturées en utilisant les écouteurs (233) ; et données de mouvement capturées en utilisant une montre intelligente portée par l'utilisateur.

[Fig. 1]

[Fig. 2]

[Fig. 3]

300

```
┌─────────────────────────────────────────────────┐
│  INPUT TRAINING SPECTROGRAMS FROM A SINGLE        │── 301
│  HOMOGENEOUS BREATHING AUDIO DATASET              │
└─────────────────────────────────────────────────┘
                         │
                         ▼
┌─────────────────────────────────────────────────┐
│        TRAINING AND VALIDATION DATA SPLIT         │── 303
└─────────────────────────────────────────────────┘
                         │
                         ▼
┌─────────────────────────────────────────────────┐
│           IMAGE FEATURE EXTRACTION                │── 305
│           WITH CONVOLUTIONAL LAYERS               │
└─────────────────────────────────────────────────┘
                         │
                         ▼
┌─────────────────────────────────────────────────┐
│             TRAIN FULLY CONNECTED                 │── 307
│          DNN LAYER WITH TRAINING DATA             │
└─────────────────────────────────────────────────┘
```

PREDICT RESPIRATION RATE ON VALIDATION DATA AND CALCULATE RESPIRATION RATE REGRESSION LOSS — 309

PREDICT RESPIRATION DEPTH ON VALIDATION DATA AND CALCULATE RESPIRATION DEPTH CLASSIFICATION LOSS — 311

CALCULATE HYBRID REGRESSION-CLASSIFICATION LOSS FROM TWO HETEROGENEOUS LOSSES — 313

319

UPDATE WEIGHTS

NO ◄— REACHED CONVERGENCE? —► YES 315

FINISH TRAINING — 317

[Fig. 4]

[Fig. 5]

[Fig. 6A]

601

[Fig. 6B]

602

[Fig. 6C]

603

EP 4 457 806 B1

[Fig. 7]

732

VARIOUS ACTIVITIES OF DAILY LIVING (ADL)

700

730

EARBUD CAPTURED BREATHING AUDIO AT DIFFERENT ACTIVITIES

710

TRAINING PIPELINE

712
BREATHING
AUDIO
DATASET

714
BREATHING
SPECTROGRAM

715
ADL CLUSTERED
BREATHING
SPECTROGRAM
(WALKING, SITTING, LYING, ...)

716
ADL SPECIFIC
CNN MODEL POOL

719
NEURAL ARCHITECTURE GRID SEARCH

718
CNN
MODELS
(WALKING, SITTING, LYING, ...)

720
ADL BASED MULTI-TASK LEARNING

722
RESPIRATION
RATE

724
RESPIRATION
DEPTH

726
HYBRID LOSS FUNCTION

[Fig. 8]

```
                    ┌─────────────────────────┐
                    │    INPUT ADL CLUSTERED   │
                    │   TRAINING SPECTROGRAMS  │──801        800
                    └─────────────────────────┘
                                 │
                                 ▼
                    ┌─────────────────────────┐
                    │   TRAINING AND VALIDATION│──803
                    │       DATA SPLIT         │
                    └─────────────────────────┘
                                 │
                                 ▼
                    ┌─────────────────────────┐
         ┌─────────▶│  LOAD CNN STRUCTURAL AND │──804
         │          │  TRAINING CONFIGURATION  │
         │          └─────────────────────────┘
         │                       │
         │                       ▼
         │          ┌─────────────────────────┐
         │   ┌─────▶│  IMAGE FEATURE EXTRACTION│──805
         │   │      │     WITH CNN LAYERS      │
         │   │      └─────────────────────────┘
         │   │                   │
         │   │                   ▼
         │   │      ┌─────────────────────────┐
         │   │      │ TRAIN FULLY CONNECTED DNN│──807
         │   │      │  LAYER WITH TRANING DATA │
         │   │      └─────────────────────────┘
         │   │             │           │
         │   │             ▼           ▼
         │   │  ┌──────────────┐  ┌──────────────┐
         │   │  │PREDICT       │  │PREDICT       │
         │   │  │RESPIRATION   │  │RESPIRATION   │
         │   │  │RATE ON       │  │DEPTH ON      │
         │   │  │VALIDATION    │  │VALIDATION    │
         │   │  │DATA AND      │──│DATA AND      │──811
         │   │  │CALCULATE 809 │  │CALCULATE     │
         │   │  │RESPIRATION   │  │RESPIRATION   │
         │   │  │RATE          │  │DEPTH         │
         │   │  │REGRESSION    │  │CLASSIFICATION│
         │   │  │LOSS          │  │LOSS          │
         │   │  └──────────────┘  └──────────────┘
         │   │             │           │
         │   │             ▼           ▼
         │   │      ┌─────────────────────────┐
         │   │      │    CALCULATE HYBRID      │──813
         │   │      │REGRESSION-CLASSIFICATION │
         │   │      │          LOSS            │
         │   │      └─────────────────────────┘
         │   │                   │
         │   │  817              ▼      815
         │   │ ┌──────┐  NO  ◇───────────────◇
         │   └─│UPDATE│◀─────│REACHED         │
         │     │WEIGHTS│     │CONVERGENCE?    │
         │     └──────┘      ◇───────────────◇
         │                           │ YES
         │                           ▼
         │          ┌─────────────────────────┐
         │          │   LOG VALIDATION METRIC  │──819
         │          └─────────────────────────┘
         │                           │
         │                           ▼      821
         │              NO   ◇───────────────────◇
         └──────────────────│  FINISHED ALL      │
                            │CNN CONFIGURATIONS   │
                            │  IN GRID SEARCH?    │
                            ◇───────────────────◇
                                     │ YES
                                     ▼
                    ┌─────────────────────────┐
                    │ RETURN ADL SPECIFIC CNN  │──823
                    │MODEL WITH BEST VALIDATION│
                    │         METRIC           │
                    └─────────────────────────┘
```

[Fig. 9]

EP 4 457 806 B1

[Fig. 10]

1000

START

OBTAIN AT LEAST ONE BREATHING AUDIO SAMPLE OF A
USER CAPTURED USING EARBUDS WORN BY THE USER — 1001

CONVERT THE AT LEAST ONE BREATHING AUDIO SAMPLE TO
A BREATHING SPECTROGRAM CONFIGURED AS AN IMAGE — 1003

DETERMINE AN ADL OF THE USER ASSOCIATED WITH
THE AT LEAST ONE BREATHING AUDIO SAMPLE — 1005

SELECT ONE OF MULTIPLE CNNS IN A CNN POOL AS A
TRAINED MULTI-TASK CNN BASED ON THE ADL OF THE USER — 1007

PROCESS THE BREATHING SPECTROGRAM USING THE
TRAINED MULTI-TASK CNN TO IDENTIFY A BREATHING
RATE AND A BREATHING DEPTH OF THE USER — 1009

OUTPUT THE BREATHING RATE AND THE
BREATHING DEPTH OF THE USER — 1011

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220054039 A1
- US 2019042981 A1
- WO 2020141999 A1
- US 2021146082 A1